(19) Europäisches Patentamt
European Patent Office
Office européen des brevets

(11) **EP 3 437 660 A1**

(12) **EUROPEAN PATENT APPLICATION**
published in accordance with Art. 153(4) EPC

(43) Date of publication:
06.02.2019 Bulletin 2019/06

(51) Int Cl.:
*A61K 47/42* (2017.01)   *A61K 38/46* (2006.01)
*A61K 45/00* (2006.01)

(21) Application number: 17775024.7

(22) Date of filing: 28.03.2017

(86) International application number:
PCT/JP2017/012540

(87) International publication number:
WO 2017/170487 (05.10.2017 Gazette 2017/40)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 28.03.2016 JP 2016063921

(71) Applicant: Fujifilm Corporation
Minato-ku
Tokyo 106-8620 (JP)

(72) Inventors:
• MIYOSHI, Hayato
Ashigarakami-gun
Kanagawa 258-8577 (JP)
• YOSHITANI, Toshihide
Ashigarakami-gun
Kanagawa 258-8577 (JP)
• YAMADA, Tadanori
Ashigarakami-gun
Kanagawa 258-8577 (JP)

(74) Representative: Klunker IP
Patentanwälte PartG mbB
Destouchesstraße 68
80796 München (DE)

(54) **PREPARATION, MATERIAL FOR PREPARATION, AND METHODS FOR PRODUCING THESE**

(57) Objects of the present invention are to provide a preparation, which satisfies both a sufficient drug carrying amount and a preferred decomposition rate, and a method for manufacturing the preparation and to provide a member for a preparation used in the preparation of the present invention described above and a method for manufacturing the member. According to the present invention, there are provided a preparation containing a crosslinked substance of an anionic polypeptide and a cationic polypeptide and a cationic drug; a method for manufacturing the preparation including a freezing step, a drying step, a crosslinking step, and a drug adding step; a member for a preparation formed of a crosslinked substance of an anionic polypeptide and a cationic polypeptide; and a method for manufacturing the member for a preparation.

FIG. 1

--◇-- MOLAR RATIO OF SUCCINYLATED CBE3:CBE3 = 0 : 1 (COMPARATIVE EXAMPLE)

—▲— MOLAR RATIO OF SUCCINYLATED CBE3:CBE3 = 1 : 3

—●— MOLAR RATIO OF SUCCINYLATED CBE3:CBE3 = 1 : 1

—✕— MOLAR RATIO OF SUCCINYLATED CBE3:CBE3 = 3 : 1

```
SEQUENCE LISTING
<110>  FUJIFILM Corporation
<120>  A formulation, a component for formulation, and a method for
production thereof
<130>\201@16F02100
<160>  10
<170>  PatentIn version 3.5
<210>  1
<211>  571
<212>  PRT
<213>  Recombinant
<400>  1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
            20                  25                  30
Arg Gly Asp Ala Gly Pro Lys Gly Ala Asp Gly Ala Pro Gly Ala Pro
        35                  40                  45
Gly Leu Gln Gly Met Pro Gly Glu Arg Gly Ala Ala Gly Leu Pro Gly
    50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
    130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
            180                 185                 190
Gly Ala Pro Gly Leu Gln Gly Ala Pro Gly Leu Gln Gly Met Pro Gly
            195                 200                 205
Glu Arg Gly Ala Ala Gly Leu Pro Gly Pro Lys Gly Glu Arg Gly Asp
    210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
            260                 265                 270
Asp Gly Val Arg Gly Leu Ala Gly Pro Ile Gly Pro Pro Gly Glu Arg
            275                 280                 285
Gly Ala Ala Gly Leu Pro Gly Pro Lys Gly Glu Arg Gly Asp Ala Gly
    290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
            325                 330                 335
Gly Leu Gln Gly Met Pro Gly Glu Arg Gly Ala Ala Gly Leu Pro Gly
            340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
        370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
```

```
                       405                        410                        415
          Lys Gly Ala Asp Gly Ala Pro Gly Ala Pro Gly Leu Gln Gly Met Pro
                       420                        425                        430
          Gly Glu Arg Gly Ala Ala Gly Leu Pro Gly Pro Lys Gly Glu Arg Gly
                       435                        440                        445
          Asp Ala Gly Pro Lys Gly Ala Asp Gly Ala Pro Gly Lys Asp Gly Val
               450                        455                        460
          Arg Gly Leu Ala Gly Pro Ile Gly Pro Pro Gly Glu Arg Gly Ala Ala
          465                        470                        475                        480
          Gly Leu Pro Gly Pro Lys Gly Glu Arg Gly Asp Ala Gly Pro Lys Gly
                       485                        490                        495
          Ala Asp Gly Ala Pro Gly Lys Asp Gly Val Arg Gly Leu Ala Gly Pro
                       500                        505                        510
          Ile Gly Pro Pro Gly Pro Ala Gly Ala Pro Gly Ala Pro Gly Leu Gln
               515                        520                        525
          Gly Met Pro Gly Glu Arg Gly Ala Ala Gly Leu Pro Gly Pro Lys Gly
               530                        535                        540
          Glu Arg Gly Asp Ala Gly Pro Lys Gly Ala Asp Gly Ala Pro Gly Lys
          545                        550                        555                        560
          Asp Gly Val Arg Gly Leu Ala Gly Pro Pro Gly
                       565                        570
<210>   2
<211>   4
<212>   PRT
<213>   Artificial Sequence
<220>
<223>   Description of Artificial Sequence: adhesive sequence
<400>   2
Arg Glu Asp Val
1
<210>   3
<211>   5
<212>   PRT
<213>   Artificial Sequence
<220>
<223>   Description of Artificial Sequence: adhesive sequence
<400>   3
Tyr Ile Gly Ser Arg
1               5
<210>   4
<211>   5
<212>   PRT
<213>   Artificial Sequence
<220>
<223>   Description of Artificial Sequence: adhesive sequence
<400>   4
Pro Asp Ser Gly Arg
1               5
<210>   5
<211>   7
<212>   PRT
<213>   Artificial Sequence
<220>
<223>   Description of Artificial Sequence: adhesive sequence
<400>   5
Arg Tyr Val Val Leu Pro Arg
1               5
<210>   6
<211>   6
<212>   PRT
<213>   Artificial Sequence
<220>
<223>   Description of Artificial Sequence: adhesive sequence
```

```
<400>  6
Leu Gly Thr Ile Pro Gly
1               5
<210>  7
<211>  10
<212>  PRT
<213>  Artificial Sequence
<220>
<223>  Description of Artificial Sequence: adhesive sequence
<400>  7
Arg Asn Ile Ala Glu Ile Ile Lys Asp Ile
1               5                   10
<210>  8
<211>  5
<212>  PRT
<213>  Artificial Sequence
<220>
<223>  Description of Artificial Sequence: adhesive sequence
<400>  8
Ile Lys Val Ala Val
1               5
<210>  9
<211>  4
<212>  PRT
<213>  Artificial Sequence
<220>
<223>  Description of Artificial Sequence: adhesive sequence
<400>  9
Asp Gly Glu Ala
1
<210>  10
<211>  4
<212>  PRT
<213>  Artificial Sequence
<220>
<223>  Description of Artificial Sequence: adhesive sequence
<400>  10
Glu Arg Gly Asp
1
```

```
SEQUENCE LISTING
<110>  FUJIFILM Corporation
<120>  A formulation, a component for formulation, and a method for
production thereof
<130>\201@16F02100
<160>  10
<170>  PatentIn version 3.5
<210>  1
<211>  571
<212>  PRT
<213>  Recombinant
<400>  1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
            115                 120                 125
Arg Gly Leu Ala Gly Pro Ile Gly Pro Pro Gly Pro Ala Gly Ala Pro
        130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
                195                 200                 205
Glu Arg Gly Ala Ala Gly Leu Pro Gly Pro Lys Gly Glu Arg Gly Asp
            210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
            275                 280                 285
Gly Ala Ala Gly Leu Pro Gly Pro Lys Gly Glu Arg Gly Asp Ala Gly
        290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
            325                 330                 335
Gly Leu Gln Gly Met Pro Gly Glu Arg Gly Ala Ala Gly Leu Pro Gly
            340                 345                 350
Pro Lys Gly Glu Arg Gly Asp Ala Gly Pro Lys Gly Ala Asp Gly Ala
            355                 360                 365
Pro Gly Lys Asp Gly Val Arg Gly Leu Ala Gly Pro Pro Gly Ala Pro
            370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
```

```
                         405                      410                        415
     Lys Gly Ala Asp Gly Ala Pro Gly Ala Pro Gly Leu Gln Gly Met Pro
                     420                  425                      430
     Gly Glu Arg Gly Ala Ala Gly Leu Pro Gly Pro Lys Gly Glu Arg Gly
                     435                  440                      445
     Asp Ala Gly Pro Lys Gly Ala Asp Gly Ala Pro Gly Lys Asp Gly Val
             450                  455                  460
     Arg Gly Leu Ala Gly Pro Ile Gly Pro Pro Gly Glu Arg Gly Ala Ala
     465                  470                  475                      480
     Gly Leu Pro Gly Pro Lys Gly Glu Arg Gly Asp Ala Gly Pro Lys Gly
                     485                  490                      495
     Ala Asp Gly Ala Pro Gly Lys Asp Gly Val Arg Gly Leu Ala Gly Pro
                     500                  505                      510
     Ile Gly Pro Pro Gly Pro Ala Gly Ala Pro Gly Ala Pro Gly Leu Gln
                 515                  520                  525
     Gly Met Pro Gly Glu Arg Gly Ala Ala Gly Leu Pro Gly Pro Lys Gly
             530                  535                  540
     Glu Arg Gly Asp Ala Gly Pro Lys Gly Ala Asp Gly Ala Pro Gly Lys
     545                  550                  555                      560
     Asp Gly Val Arg Gly Leu Ala Gly Pro Pro Gly
                     565                  570
```

<210>   2
<211>   4
<212>   PRT
<213>   Artificial Sequence
<220>
<223>   Description of Artificial Sequence: adhesive sequence
<400>   2
Arg Glu Asp Val
1
<210>   3
<211>   5
<212>   PRT
<213>   Artificial Sequence
<220>
<223>   Description of Artificial Sequence: adhesive sequence
<400>   3
Tyr Ile Gly Ser Arg
1                   5
<210>   4
<211>   5
<212>   PRT
<213>   Artificial Sequence
<220>
<223>   Description of Artificial Sequence: adhesive sequence
<400>   4
Pro Asp Ser Gly Arg
1                   5
<210>   5
<211>   7
<212>   PRT
<213>   Artificial Sequence
<220>
<223>   Description of Artificial Sequence: adhesive sequence
<400>   5
Arg Tyr Val Val Leu Pro Arg
1                   5
<210>   6
<211>   6
<212>   PRT
<213>   Artificial Sequence
<220>
<223>   Description of Artificial Sequence: adhesive sequence

```
<400>  6
Leu Gly Thr Ile Pro Gly
1               5
<210>  7
<211>  10
<212>  PRT
<213>  Artificial Sequence
<220>
<223>  Description of Artificial Sequence: adhesive sequence
<400>  7
Arg Asn Ile Ala Glu Ile Ile Lys Asp Ile
1               5                   10
<210>  8
<211>  5
<212>  PRT
<213>  Artificial Sequence
<220>
<223>  Description of Artificial Sequence: adhesive sequence
<400>  8
Ile Lys Val Ala Val
1               5
<210>  9
<211>  4
<212>  PRT
<213>  Artificial Sequence
<220>
<223>  Description of Artificial Sequence: adhesive sequence
<400>  9
Asp Gly Glu Ala
1
<210>  10
<211>  4
<212>  PRT
<213>  Artificial Sequence
<220>
<223>  Description of Artificial Sequence: adhesive sequence
<400>  10
Glu Arg Gly Asp
1
```

**Description**

**BACKGROUND OF THE INVENTION**

1. Field of the Invention

The present invention relates to a preparation containing an anionic polypeptide, a cationic polypeptide, and a cationic drug, and a member for a preparation containing an anionic polypeptide and a cationic polypeptide. The present invention also relates to a method for manufacturing the preparation and a method for manufacturing the member for a preparation. The preparation and the member for a preparation of the present invention can be used in pharmaceutical products.

2. Description of the Related Art

Making a preparation by incorporating a drug into a member for a preparation is widely practiced. For example, as a method for causing a drug to be slowly released, a method is known in which a drug is incorporated into a member for a preparation so as to manufacture a slow-release preparation. Patent Literature 1 describes a composition for blocking blood vessels containing a fibrous member, a member capable of slowly releasing a cell growth factor, and a cell growth factor. Patent Literature 2 describes a slow-release pharmaceutical composition containing a ternary complex constituted with interleukin-11, an anionic polymer, and a cationic polymer. Patent Literature 2 describes that hyaluronic acid or the like is used as the anionic polymer, and protamine or the like is used as the cationic polymer.
Patent Literature 3 describes a method for manufacturing microparticles by simultaneously nebulizing an active component, a matrix-forming component A, and a matrix-forming component B capable of binding to the matrix-forming component A. Patent Literature 3 describes that a compound having a cationic dissociable group is used as the matrix-forming component A, and a compound having an anionic dissociable group is used as the matrix-forming component B.

Prior Art Literatures

Patent Literatures

Patent Literature 1: JP2003-48841A
Patent Literature 2: JP2006-96751A
Patent Literature 3: WO2015/025979A

**SUMMARY OF THE INVENTION**

Although a slow-release preparation containing a drug, an anionic polymer, and a cationic polymer is known as described above, a preparation satisfying both a sufficient drug carrying amount and a preferred decomposition rate has not been examined. An object of the present invention is to provide a preparation satisfying both a sufficient drug carrying amount and a preferred decomposition rate and a method for manufacturing the preparation. Another object of the present invention is to provide a member for a preparation that is for used in the preparation of the present invention and a method for manufacturing the member.
In order to achieve the aforementioned objects, the inventors of the present invention conducted intensive examinations. As a result, the inventors have found that by using a crosslinked substance, which is obtained by crosslinking an anionic polypeptide and a cationic polypeptide, as a member for a preparation containing a cationic drug, a preparation for achieving the aforementioned objects can be provided, and have accomplished the present invention.
That is, according to the present invention, the following inventions are provided.

[1] A preparation comprising a crosslinked substance of an anionic polypeptide and a cationic polypeptide, and a cationic drug.
[2] The preparation described in [1] that is for local administration.
[3] The preparation described in [1] or [2], in which a molar ratio of the anionic polypeptide to the cationic polypeptide is 1 to 3 to 3 to 1.
[4] The preparation described in any one of [1] to [3], in which each of the anionic polypeptide and the cationic polypeptide is a gene recombinant, which has an amino acid sequence derived from a partial amino acid sequence of collagen, or a chemical modification product thereof.

[5] The preparation described in [4], in which the anionic polypeptide is a chemical modification product of a cationic polypeptide, and the cationic polypeptide is a chemical modification product of an anionic polypeptide.

[6] The preparation described in [4] or [5], in which the gene recombinant having an amino acid sequence derived from a partial amino acid sequence of collagen has a repeating sequence represented by Gly-X-Y, X and Y each independently represent any amino acid, a plurality of sequences represented by Gly-X-Y may be the same as or different from each other, and a molecular weight of the gene recombinant is equal to or greater than 10 kDa and equal to or smaller than 90 kDa.

[7] The preparation described in any one of [4] to [6], in which the gene recombinant having an amino acid sequence derived from a partial amino acid sequence of collagen is represented by the following formula.

Formula: A-[(Gly-X-Y)n]m-B

In the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.

[8] The preparation described in any one of [4] to [7], in which the gene recombinant having an amino acid sequence derived from a partial amino acid sequence of collagen has (1) amino acid sequence described in SEQ ID NO: 1 or (2) amino acid sequence which shares a sequence identity equal to or higher than 80% with the amino acid sequence described in SEQ ID NO: 1 and has a function of carrying the cationic drug.

[9] The preparation described in any one of [1] to [8], in which the crosslinked substance of an anionic polypeptide and a cationic polypeptide is a crosslinked substance obtained by a heat treatment.

[10] The preparation described in any one of [1] to [9], in which the cationic drug is a polypeptide.

[11] A member for a preparation comprising a crosslinked substance of an anionic polypeptide and a cationic polypeptide.

[12] The member for a preparation described in [11], in which a molar ratio of the anionic polypeptide to the cationic polypeptide is 1 to 3 to 3 to 1.

[13] The member for a preparation described in [11] or [12], in which each of the anionic polypeptide and the cationic polypeptide is a gene recombinant, which has an amino acid sequence derived from a partial amino acid sequence of collagen, or a chemical modification product thereof.

[14] The member for a preparation described in [13], in which the anionic polypeptide is a chemical modification product of a cationic polypeptide, and the cationic polypeptide is a chemical modification product of an anionic polypeptide.

[15] The member for a preparation described in [13] or [14], in which the gene recombinant having an amino acid sequence derived from a partial amino acid sequence of collagen has a repeating sequence represented by Gly-X-Y, X and Y each independently represent any amino acid, a plurality of sequences represented by Gly-X-Y may be the same as or different from each other, and a molecular weight of the gene recombinant is equal to or greater than 10 kDa and equal to or smaller than 90 kDa.

[16] The member for a preparation described in any one of [13] to [15], in which the gene recombinant having an amino acid sequence derived from a partial amino acid sequence of collagen is represented by the following formula.

Formula: A-[(Gly-X-Y)n]m-B

In the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.

[17] The member for a preparation described in any one of [13] to [16], in which the gene recombinant having an amino acid sequence derived from a partial amino acid sequence of collagen has (1) amino acid sequence described in SEQ ID NO: 1 or (2) amino acid sequence which shares a sequence identity equal to or higher than 80% with the amino acid sequence described in SEQ ID NO: 1 and has a function of carrying the cationic drug.

[18] The member for a preparation described in any one of [11] to [17], in which the crosslinked substance of an anionic polypeptide and a cationic polypeptide is a crosslinked substance obtained by a heat treatment.

[19] A method for manufacturing the member for a preparation described in any one of [11] to [18], comprising a freezing step of freezing a solution containing an anionic polypeptide and a cationic polypeptide; a drying step of drying a frozen substance obtained by the freezing step; and a crosslinking step of crosslinking the anionic polypeptide and the cationic polypeptide of a dried substance obtained by the drying step.

[20] A method for manufacturing the preparation described in any one of [1] to [10], comprising a freezing step of freezing a solution containing an anionic polypeptide and a cationic polypeptide; a drying step of drying a frozen substance obtained by the freezing step; a crosslinking step of crosslinking the anionic polypeptide and the cationic polypeptide of a dried substance obtained by the drying step; and a drug adding step of incorporating a cationic drug into a crosslinked substance of the anionic polypeptide and the cationic polypeptide obtained by the crosslinking step.

The preparation of the present invention can carry a sufficient amount of a drug and exhibits a preferred decomposition rate. According to the member for a preparation of the present invention and the method of the present invention, it is possible to manufacture a preparation which can carry a sufficient amount of a drug and exhibits a preferred decomposition rate.

## BRIEF DESCRIPTION OF THE DRAWINGS

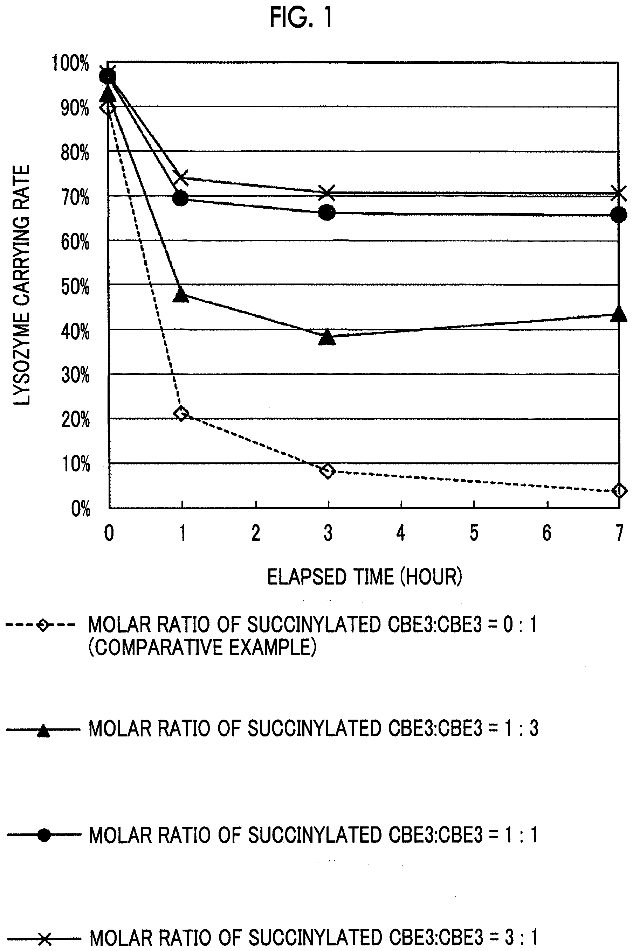

Fig. 1 shows a lysozyme carrying rate of a preparation at each elapsed time point.

## DESCRIPTION OF THE PREFERRED EMBODIMENTS

Hereinafter, embodiments of the present invention will be specifically described.

[Preparation]

The preparation according to the present invention is a preparation containing a crosslinked substance of an anionic polypeptide and a cationic polypeptide and a cationic drug.
In the present invention, it was found that by using a crosslinked substance of an anionic polypeptide and a cationic polypeptide and combining the crosslinked substance with a cationic drug, both a sufficient drug carrying amount and a preferred decomposition rate can be achieved. According to JP2003-48841A, partially crosslinked gelatin is used as a slow-release member of a cell growth factor, but the partially crosslinked gelatin is not a slow-release member formed of a cationic polypeptide and an anionic polypeptide. Although JP2006-96751A describes a slow-release preparation containing a ternary complex constituted with interleukin-11, an anionic polymer, and a cationic polymer, the preparation is not a crosslinked substance of an anionic polymer and a cationic polymer. Although WO2015/025979A describes a method for manufacturing microparticles by using an active component, a matrix-forming component A, and a matrix-forming component B, this is not a method for forming a crosslinked substance of the matrix-forming component A and the matrix-forming component B.
As will be shown in examples and comparative examples which will be described later in the present specification, it was confirmed that by using a crosslinked substance of an anionic polypeptide and a cationic polypeptide, a preparation which exhibits a sufficiently high acidolysis residual rate, that is, a preferred decomposition rate can be provided. In the technique of the related art, the object of achieving both the sufficient drug carrying amount and the preferred decomposition rate has not been sufficiently examined. The characteristic of the present invention is that both the sufficient drug carrying amount and the preferred decomposition rate can be achieved by the constitution in which the crosslinked substance of an anionic polypeptide and a cationic polypeptide and the cationic drug are used.

(1) Anionic polypeptide

The anionic polypeptide means a polypeptide having a property of being negatively charged under a physiological condition (pH 7.4).
Examples of the anionic polypeptide include alkali-treated gelatin, polyglutamic acid, polyaspartic acid, bovine serum albumin, and a gene recombinant having an amino acid sequence derived from a partial amino acid sequence of these.
As the anionic polypeptide, a polypeptide may be used which is obtained by modifying a cationic polypeptide (for example, acid-treated gelatin, collagen, protamine, polyarginine, a gene recombinant having an amino acid sequence derived from a partial amino acid sequence of these, or the like) into an anionic polypeptide by a chemical reaction. Examples of the chemical reaction include a method of modifying a cationic amino group into an anionic carboxyl group by a treatment using succinic anhydride or maleic anhydride, a method of modifying a cationic amino group into an uncharged functional group by using acetic anhydride, N-hydroxysuccinimide acetate, N-acetylsuccinimide, phenyl isocyanate, or acetaldehyde. As described above, a polypeptide obtained by modifying a cationic polypeptide into an anionic polypeptide by a chemical reaction is referred to as a chemical modification product of the cationic polypeptide.
The anionic polypeptide is preferably a polypeptide obtained by modifying a cationic polypeptide (for example, acid-treated gelatin, collagen, or a gene recombinant having an amino acid sequence derived from a partial amino acid sequence of these) into an anionic polypeptide by a chemical reaction, and more preferably a polypeptide obtained by modifying a gene recombinant having an amino acid sequence derived from a partial amino acid sequence of collagen into an anionic polypeptide by a treatment using succinic anhydride.

"Gene recombinant having an amino acid sequence derived from a partial amino acid sequence of collagen" will be described later.

The anionic polypeptide may be any of a naturally occurring peptide, a recombinant peptide, and a chemically synthesized peptide.

In the present invention, a chemically synthesized peptide means an artificially synthesized peptide. The synthesis of a peptide such as gelatin may be solid-phase synthesis or liquid-phase synthesis, and is preferably solid-phase synthesis. The solid-phase synthesis of a peptide is known to those skilled in the related art, and examples thereof include an Fmoc group synthesis method in which a Fluorenyl-Methoxy-Carbonyl group (Fmoc group) is used for protecting an amino group, a Boc group synthesis method in which a tert-Butyl Oxy Carbonyl group (Boc group) is used for protecting an amino group, and the like.

(2) Cationic polypeptide

The cationic polypeptide means a polypeptide having a property of being positively charged under a physiological condition (pH 7.4).

Examples of the cationic polypeptide include acid-treated gelatin, collagen, protamine, polyarginine, and a gene recombinant having an amino acid sequence derived from a partial amino acid sequence of these. Among these, acid-treated gelatin, collagen, and a gene recombinant having an amino acid sequence derived from a partial amino acid sequence of these are preferable, and a gene recombinant having an amino acid sequence derived from a partial amino acid sequence of collagen is more preferable.

As the cationic polypeptide, a polypeptide may be used which is obtained by modifying an anionic polypeptide into a cationic polypeptide by a chemical reaction. Examples of the chemical reaction include a method of modifying an anionic carboxyl group into a cationic amino group by using a diamine such as ethylenediamine, a method of modifying an anionic carboxyl group into an uncharged functional group by using an aminothiol such as 2-aminoethanethiol or an amino alcohol such as 2-amino alcohol, and a method of modifying an anionic carboxyl group into an uncharged functional group through reductive amination by using 5-ethyl-methylpyridine borane or the like. As described above, the polypeptide obtained by modifying an anionic polypeptide into a cationic polypeptide by a chemical reaction is referred to as a chemical modification product of the anionic polypeptide.

"Gene recombinant having an amino acid sequence derived from a partial amino acid sequence of collagen" will be described later.

The cationic polypeptide may be any of a naturally occurring peptide, a recombinant peptide, and a chemically synthesized peptide.

(3) Ratio between anionic polypeptide and cationic polypeptide

A molar ratio of the anionic polypeptide to the cationic polypeptide is not particularly limited unless the effects of the present invention are impaired. The molar ratio is preferably 1 to 5 to 5 to 1, more preferably 1 to 3 to 3 to 1, even more preferably 1 to 2 to 2 to 1, and particularly preferably 1 to 1.

(4) Gene recombinant having amino acid sequence derived from partial amino acid sequence of collagen

The gene recombinant having an amino acid sequence derived from a partial amino acid sequence of collagen is a polypeptide or a protein-like substance which is prepared by a gene recombination technology and has an amino acid sequence analogous to collagen or gelatin. In the present specification, the gene recombinant having an amino acid sequence derived from a partial amino acid sequence of collagen is referred to as recombinant gelatin as well.

It is preferable that the recombinant gelatin has a repeating sequence represented by Gly-X-Y (X and Y each independently represent any amino acid) which is a characteristic of collagen. A plurality of sequences represented by Gly-X-Y may be the same as or different from each other.

As the recombinant gelatin, for example, it is possible to use those described in EP1014176, US6992172A, WO2004/85473A, WO2008/103041A, and the like. However, the recombinant gelatin is not limited thereto. As the recombinant gelatin used in the present invention, recombinant gelatin of the following aspects is preferable.

The recombinant gelatin exhibits excellent biocompatibility due to the performance inherent to the natural gelatin, does not carry a risk of causing bovine spongiform encephalopathy (BSE) because the recombinant gelatin is not a naturally occurring substance, and is excellently noninfectious. Furthermore, because the recombinant gelatin is more homogenous than the natural gelatin and has a predetermined sequence, it is possible to accurately design the recombinant gelatin while reducing the variation in strength and decomposition properties by crosslinking and the like.

The molecular weight of the recombinant gelatin is not particularly limited, but is preferably equal to or greater than 2,000 to equal to or smaller than 100,000 (equal to or greater than 2 kDa (kilodaltons) and equal to or smaller than 100 kDa), more preferably equal to or greater than 2,500 and equal to or smaller than 95,000 (equal to or greater than 2.5 kDa and equal to or smaller than 95 kDa), even more preferably equal to or greater than 5,000 and equal to or smaller than 90,000 (equal to or greater than 5 kDa and equal to or smaller than 90 kDa), and most preferably equal to or greater than 10,000 and equal to or smaller than 90,000 (equal to or greater than 10 kDa and equal to or smaller than 90 kDa).

It is preferable that the recombinant gelatin has a repeating sequence represented by Gly-X-Y which is a characteristic of collagen. A plurality of sequences represented by Gly-X-Y may be the same as or different from each other. In Gly-X-Y, Gly represents glycine, and each of X and Y represents any amino acid (preferably any amino acid other than glycine). The sequence represented by Gly-X-Y which is a characteristic of collagen is a partial structure extremely unique in the composition and sequence of the amino acid of gelatin·collagen compared to other proteins. Glycine takes up about 1/3 of this portion and is one of the three repeating amino acids in the amino acid sequence. Glycine is the simplest amino acid, and the disposition of the molecular chain thereof is restricted less. Furthermore, glycine makes a big contribution to the regeneration of a helix structure at the time of gelation. The amino acids represented by X and Y contain a large amount of imino acids (proline and oxyproline), and the content of the imino acids is preferably 10% to 45% of the total content of the amino acids. In the sequence of the recombinant gelatin, the proportion of amino acids constituted with the repeating structure of Gly-X-Y is preferably equal to or higher than 80%, more preferably equal to or higher than 95%, and most preferably equal to or higher than 99%.

Generally, gelatin contains charged polar amino acids and uncharged polar amino acids at 1:1. The polar amino acids specifically refer to cysteine, aspartic acid, glutamic acid, histidine, lysine, asparagine, glutamine, serine, threonine, tyrosine, and arginine. Among these, uncharged polar amino acids refer to cysteine, asparagine, glutamine, serine, threonine, and tyrosine. In the recombinant gelatin used in the present invention, the proportion of the polar amino acids in all the constituent amino acids is 10% to 40% and preferably 20% to 30%. The proportion of the uncharged amino acids in the polar amino acids is preferably equal to or higher than 5% and less than 20%, and more preferably equal to or higher than 5% and less than 10%. Furthermore, it is preferable that gelatin does not contain one amino acid and preferably does not contain two or more amino acids among serine, threonine, asparagine, tyrosine, and cysteine in the sequence thereof.

Generally, regarding polypeptides, minimal amino acid sequence functioning as cell adhesion signals are known (for example, "Pathophysiology" published from Nagai Publishing Co., Ltd., Vol. 9, No. 7 (1990), p. 527). The recombinant gelatin used in the present invention may have two or more cell adhesion signals in one molecule. Specifically, as such sequences, an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence represented by letter notation of amino acids are preferable, because these sequences enable the adhesion of many kinds of cells. Among these, an RGD sequence, a YIGSR sequence, a PDSGR sequence, an LGTIPG sequence, an IKVAV sequence, and an HAV sequence are more preferable, and an RGD sequence is particularly preferable. Among RGD sequences, an ERGD sequence is preferable.

Regarding the disposition of the RGD sequence in the recombinant gelatin used in the present invention, it is preferable that the number of amino acids between RGD sequences varies in a range of 0 to 100 and preferably varies in a range of 25 to 60.

The number of minimal amino acid sequences described above contained in one molecule of the protein is preferably 3 to 50, more preferably 4 to 30, particularly preferably 5 to 20, and most preferably 12.

In the recombinant gelatin used in the present invention, the ratio of the RGD motif to the total number of amino acids is preferably at least 0.4%. In a case where the recombinant gelatin contains 350 or more amino acids, it is preferable that each stretch of the 350 amino acids contains at least one RGD motif. The ratio of the RGD motif to the total number of amino acids is more preferably at least 0.6%, even more preferably at least 0.8%, still more preferably at least 1.0%, particularly preferably at least 1.2%, and most preferably at least 1.5%. The number of RDG motifs in the recombinant peptide is preferably at least 4, more preferably 6, even more preferably 8, and particularly preferably equal to or greater than 12 and equal to or smaller than 16 per 250 amino acids. The ratio of 0.4% of the RDG motif means that the recombinant gelatin contains at least one RGD sequence per 250 amino acids. The number of RDG motifs is an integer. Therefore, in order to satisfy the characteristic of 0.4%, gelatin constituted with 251 amino acids has to contain at least two RDG sequences. The recombinant gelatin of the present invention preferably contains at least two RGD sequences per 250 amino acids, more preferably contains at least three RGD sequences per 250 amino acids, and even more preferably contains at least four RGD sequences per 250 amino acids. In another aspect, the recombinant gelatin of the present invention contains at least four RGD motifs, preferably contains six RGD motifs, more preferably contains eight RGD motifs, and even more preferably contains two to sixteen RGD motifs.

The recombinant gelatin may be partially hydrolyzed.

It is preferable that the recombinant gelatin used in the present invention is represented by Formula 1: A-[(Gly-X-Y)$_n$]$_m$-B. n X's each independently represent any amino acid, and n Y's each independently represent any amino acid. m is preferably an integer of 2 to 10, and more preferably an integer of 3 to 5. n is preferably an integer of 3 to 100, more preferably an integer of 15 to 70, and most preferably an integer of 50 to 65. A represents any amino acid or any amino acid sequence, and B represents any amino acid or any amino acid sequence. n sequences represented by Gly-X-Y may be the same as or different from each other.

The recombinant gelatin used in the present invention is more preferably represented by Formula: Gly-Ala-Pro-[(Gly-X-Y)$_{63}$]$_3$-Gly (in the formula, 63 X's each independently represent any amino acid, 63 Y's each independently represent any amino acid, and 63 sequences represented by Gly-X-Y may be the same as or different from each other).

It is preferable that a plurality of sequence units of naturally occurring collagen are bonded to the repeating unit. The naturally occurring collagen is not limited as long as it exists in the nature. As the naturally occurring collagen, type I, type II, type III, type IV, or type V collagen is preferable, and type I, type II, or type III collagen is more preferable. According to another aspect, the collagen is preferably derived from human beings, cows, pigs, mice, or rats, and more preferably derived from human beings.

The isoelectric point of the recombinant gelatin used in the present invention is preferably 5 to 10, more preferably 6 to 10, and even more preferably 7 to 9.5. The isoelectric point of the recombinant gelatin can be determined by measuring pH after allowing a 1% by mass gelatin solution and cation and anion exchange resins to pass through a mixed crystal column as described in isoelectric focusing electrophoresis (see Maxey, C. R. (1976); Phintogr. Gelatin 2, Editor Cox, P. J. Academic, London, Engl.).

It is preferable that the recombinant gelatin is deaminated.

It is preferable that the recombinant gelatin does not have a telopeptide.

It is preferable that the recombinant gelatin is a substantially pure polypeptide prepared by nucleic acids encoding amino acid sequences.

The recombinant gelatin is particularly preferably any of (1) peptide having an amino acid sequence described in SEQ ID NO: 1; (2) peptide having an amino acid sequence, which is obtained by the deletion, substitution, or addition of one or several amino acids in the amino acid sequence described in SEQ ID NO: 1, and functioning to carry a cationic drug; and (3) peptide having an amino acid sequence, which shares a sequence identity equal to or higher than 80% (more preferably equal to or higher than 90%, particularly preferably equal to or higher than 95%, and most preferably equal to or higher than 98%) with the amino acid sequence described in SEQ ID NO: 1, and functioning to carry a cationic drug.

In the present invention, the sequence identity refers to a value calculated by the following equation.

$$\% \text{ Sequence identity} = [(\text{number of same residues})/(\text{alignment length})] \times 100$$

The sequence identity shared between two amino acid sequences can be determined by any method known to those skilled in the related art by using a Basic Local Alignment Search Tool (BLAST) program (J. Mol. Biol. 215:403-410, 1990) and the like.

"One or several amino acids" in "amino acid sequence which is obtained by the deletion, substitution, or addition of one or several amino acids" preferably means 1 to 20 amino acids, more preferably means 1 to 10 amino acids, even more preferably means 1 to 5 amino acids, and particularly preferably means 1 to 3 amino acids.

The recombinant gelatin can be manufactured by gene recombination technologies known to those skilled in the related art. For example, the recombinant gelatin can be manufactured based on the methods described in EP1014176A2, US6992172A, WO2004/85473A, WO2008/103041A, and the like. Specifically, genes encoding an amino acid sequence of a predetermined recombinant gelatin are obtained and incorporated into an expression vector, thereby preparing a recombinant expression vector. The recombinant expression vector is introduced into an appropriate host, thereby preparing a transformant. By culturing the obtained transformant in an appropriate medium, recombinant gelatin is produced. Therefore, by collecting the recombinant gelatin produced from the culture, the recombinant gelatin used in the present invention can be prepared.

"1/IOB" value which is a hydrophilicity value of the recombinant gelatin is preferably 0 to 1.0, more preferably 0 to 0.6, and even more preferably 0 to 0.4. IOB is an index of hydropathicity based on the organic conception diagram showing polarity/non-polarity of organic compounds that was suggested by Atsushi Fujita. Details of IOB are described, for example, in "Pharmaceutical Bulletin", vol. 2, 2, pp. 163-173 (1954), "Scope of Chemistry", vol. 11, 10, pp.719-725 (1957), "Fragrance Journal", vol. 50, pp. 79-82 (1981), and the like. In brief, methane ($CH_4$) is regarded as the origin of all organic compounds, all of other compounds are regarded as derivatives of methane, and a certain numerical value is assigned to the number of carbon atoms, the substituent, the transformative portion, the ring, and the like of the compounds. The scores are added up so as to determine an organic value (OV) and an inorganic value (IV), and a graph is created by plotting the organic value on the X-axis and the inorganic value on the Y-axis. IOB in the organic conception diagram refers to a ratio of the inorganic value (IV) to the organic value (OV) in the organic conception diagram, that is, "inorganic value (IV)/organic value (OV)". For details of the organic conception diagram, see "New Edition of Organic Conception Diagram-Fundamentals and Applications-" (Yoshiki Koda et al., SANKYO SHUPPAN Co., Ltd., 2008). In the present specification, hydropathicity is represented by "1/IOB" value which is the reciprocal of IOB showing that the smaller the "1/IOB" value (the closer the "1/IOB" value to 0), the higher the hydrophilicity.

In a case where the "1/IOB" value of the recombinant gelatin is within the above range, the hydrophilicity is high, and the water absorbing properties are improved.

A hydropathicity index of the recombinant gelatin represented by a Grand average of hydropathicity (GRAVY) value is preferably equal to or lower than 0.3 and equal to or higher than -9.0, and more preferably equal to or lower than 0.0 and equal to or higher than -7.0. The Grand average of hydropathicity (GRAVY) value can be obtained by the methods in "Gasteiger E., Hoogland C., Gattiker A., Duvaud S., Wilkins M.R., Appel R.D., Bairoch A.;Protein Identification and Analysis Tools on the ExPASy Server;(In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788(2003)".

In a case where the GRAVY value of the recombinant gelatin is within the above range, the hydrophilicity is high, and the water absorbing properties are improved.

(5) Crosslinked substance of anionic polypeptide and cationic polypeptide

In the present invention, an anionic polypeptide and a cationic polypeptide are crosslinked. By using the crosslinked substance of the anionic polypeptide and the cationic polypeptide, it is possible to provide a preparation which exhibits a sufficiently high acidolysis residual rate and a preferred decomposition rate.

As general crosslinking methods, thermal crosslinking, crosslinking by aldehydes (for example, formaldehyde, glutaraldehyde, and the like), crosslinking by a condensation agent (carbodiimide, cyanamide, and the like), enzymatic crosslinking, optical crosslinking, ultraviolet crosslinking, hydrophobic interaction, hydrogen bonding, ionic interaction, and the like are known. In the present invention, these crosslinking methods can also be used. As the crosslinking method used in the present invention, thermal crosslinking, ultraviolet crosslinking, or enzymatic crosslinking are more preferable, and thermal crosslinking is particularly preferable.

In a case where the crosslinking is performed using an enzyme, the enzyme is not particularly limited as long as it functions to crosslink polymer materials with each other. The crosslinking can be performed preferably using transglutaminase and laccase, and most preferably using transglutaminase. Specific examples of proteins enzymatically crosslinked by transglutaminase are not particularly limited as long as they are proteins having a lysine residue and a glutamine residue. The transglutaminase may be derived from mammals or from microorganisms. Specifically, examples thereof include an ACTIVA series manufactured by AJINOMOTO CO., INC., mammal-derived transglutaminase marketed as a reagent such as guinea pig liver-derived transglutaminase, goat-derived transglutaminase, and rabbit-derived transglutaminase manufactured by Oriental Yeast Co., ltd., Upstate USA Inc., Biodesign International, and the like, human-derived blood coagulation factors (Factor XIIIa, Haematologic Technologies, Inc.), and the like.

The reaction temperature at the time of performing the crosslinking (for example, thermal crosslinking) is not particularly limited as long as crosslinking can be carried out. However, the reaction temperature is preferably -100°C to 500°C, more preferably 0°C to 300°C, even more preferably 50°C to 300°C, even more preferably 100°C to 250°C, and particularly preferably 120°C to 200°C.

The reaction time at the time of performing the crosslinking (for example, thermal crosslinking) is not particularly limited, but is generally 1 hour to 72 hours, preferably 2 hours to 48 hours, and more preferably 3 hours to 36 hours.

In the present invention, a degree of crosslinking (number of crosslinks per molecule) of the crosslinked substance is not particularly limited, but is preferably equal to or higher than 0.5, more preferably equal to or higher than 0.5 and equal to or lower than 30, even more preferably equal to or higher than 1 and equal to or lower than 25, and particularly preferably equal to or higher than 1 and equal to or lower than 20.

The method for measuring the degree of crosslinking (number of crosslinks per molecule) of the crosslinked substance is not particularly limited. For example, the degree of crosslinking can be measured by 2,4,6-trinitrobenzenesulfonic acid (TNBS) method. Specifically, the crosslinked substance, an aqueous $NaHCO_3$ solution, and an aqueous TNBS solution are mixed together and reacted for 3 hours at 37°C, and then the reaction is stopped, thereby preparing a sample. Furthermore, the crosslinked substance, an aqueous $NaHCO_3$ solution, and an aqueous TNBS solution are mixed together, and then the reaction is stopped immediately, thereby preparing a blank. The sample and the blank are diluted with pure water, the absorbance (345 nm) thereof is measured, and from the following (Formula 1) and (Formula 2), the degree of crosslinking (number of crosslinks per molecule) can be calculated.

$$\text{(Formula 1)} \quad (As - Ab)/14600 \times V/w$$

(Formula 1) shows the amount of lysine (molar equivalent) per 1 g of the crosslinked substance.

(In the formula, As represents the absorbance of the sample, Ab represents the absorbance of the blank, V represents the amount of the reaction solution (g), and w represents the mass (mg) of the crosslinked substance.)

$$\text{(Formula 2)} \quad 1 - (\text{sample (Formula 2)}/\text{uncrosslinked polypeptide (Formula 2)}) \times 34$$

(Formula 2) shows the number of crosslinks per molecule.

(6) Cationic drug

The cationic drug means a drug having a property of being positively charged under a physiological condition (pH 7.4). As the cationic drug, a polypeptide is preferable. The polypeptide is not particularly limited, and examples thereof include a cell growth factor, an enzyme, cytokine, and the like.

The polypeptide as the cationic drug means a substance formed of a plurality of amino acids polymerized by a peptide bond. The polypeptide is preferably a substance formed of 10 or more amino acids polymerized. A protein is a sort of polypeptide.

The cell growth factor means an endogenous protein which stimulates the growth or differentiation of a specific cell in the body of an animal. Examples of the cell growth factor include a platelet-derived growth factor (PDGF), a transforming growth factor (TGF), a vascular endothelial growth factor (VEGF), a connective tissue growth factor (CTGF), a hepatocyte growth factor (HGF), a brain-derived neurotrophic factor (BDNF), an insulin-like growth factor (IGF), a nerve growth factor (NGF), a glial cell line-derived neurotrophic factor (GDNF), and the like.

The enzyme means a molecule functioning as a catalyst for a chemical reaction occurring in a biological body. Examples of the enzyme include lysozyme, trypsin, $\alpha$-chymotrypsin, urokinase, elastase, cytochrome C, bromelain, papain, and the like.

The cytokine means a protein which is secreted from cells of immune system, and cells transmitting unspecified information are target cells of the cytokine. Examples of the cytokine include interferon, interleukin, erythropoietin, and the like.

The content of the cationic drug in the preparation of the present invention is not particularly limited as long as the effects of the present invention are not impaired. The content of the cationic drug with respect to the mass of the crosslinked substance of the anionic polypeptide and the cationic polypeptide is generally 0.001% by mass to 10% by mass, preferably 0.01% by mass to 10% by mass, and more preferably 0.01% by mass to 5% by mass.

(7) How to use preparation

The preparation of the present invention contains the cationic drug, and can be used as a medication which is an agent for treating diseases. The preparation of the present invention is preferably a slow-release preparation, and can release the cationic drug at an appropriate rate.

The slow-release preparation is generally used for the purpose of causing the drug concentration to remain constant for a long period of time, and means a preparation from which a drug is slowly eluted.

The dose, the mode of administration, and the formulation form of the preparation of the present invention can be appropriately determined according to the purpose of use. For example, the preparation of the present invention may be directly administered to a target legion in a biological body, or may be administered, for example, by injection, application, and the like by being suspended in a liquid excipient such as aqueous solvents including distilled water for injection, physiological saline for injection, and a buffer (based on phosphate, citrate, and the like) having a pH of 5 to 8. Furthermore, the preparation of the present invention may be applied after being made into an ointment, a gel, a cream, and the like by being mixed with an appropriate excipient.

The mode of administration of the preparation of the present invention may be oral administration, parenteral administration (for example, intravenous administration, intramuscular administration, subcutaneous administration, intradermal administration, and the like), or local administration. For example, in order to treat cardiac diseases and the like, the preparation may be directed injected into the cardiac muscle from the ventricular cavity by using a catheter, or may be locally released or applied to a stenosed or obstructed portion in the coronary artery by using a catheter.

Examples of the formulation form include oral administration agents such as a tablet, powder, a capsule, granules, an extract, and a syrup, parenteral administration agents such as an injection (for example, an intravenous injection, an intramuscular injection, a subcutaneous injection, and an intradermal injection), and the like. In a case where the preparation is given by local administration, the form of the preparation of the present invention is not particularly specified, but examples thereof include a sponge, a film, non-woven cloth, fiber (tube), particles, mesh, and the like.

The dose of the preparation of the present invention is not particularly limited as long as the effects of the present invention are not impaired, but is generally equal to or smaller than 10 g, preferably equal to or smaller than 5 g, and more preferably equal to or smaller than 1 g.

The preparation of the present invention can be formulated by the methods known to those skilled in the related art. For example, in a case where a liquid is used as a carrier for the preparation, the preparation may be dissolved or dispersed, and in a case where powder is used as a carrier for the preparation, the preparation may be mixed with or adsorbed onto the powder. In addition, if necessary, pharmacologically accepted additives (for example, a preservative, a stabilizer, an antioxidant, an excipient, a binder, a disintegrant, a moistening agent, a lubricant, a colorant, an aromatic, a flavoring agent, a coating, a suspending agent, an emulsifier, a solubilizing agent, a buffering agent, a tonicity agent, a plasticizer, a surfactant, a soothing agent, and the like) can be incorporated into the preparation.

[Member for preparation]

As described above, the preparation of the present invention contains a crosslinked substance of an anionic polypeptide and cationic polypeptide and a cationic drug. In the preparation of the present invention, the crosslinked substance of an anionic polypeptide and a cationic polypeptide is used as a member for achieving both of a sufficient drug carrying amount and a preferred decomposition rate. That is, according to the present invention, a member for a preparation formed of a crosslinked substance of an anionic polypeptide and a cationic polypeptide is also provided. Details of the crosslinked substance of an anionic polypeptide and a cationic polypeptide constituting the member for a preparation of the present invention and a preferred scope thereof are as described above in the present specification.

[Method for manufacturing member for preparation formed of crosslinked substance of anionic polypeptide and cationic polypeptide]

The present invention also provides a method for manufacturing the member for a preparation of the present invention, including a freezing step of freezing a solution containing an anionic polypeptide and a cationic polypeptide; a drying step of drying a frozen substance obtained by the freezing step; and a crosslinking step of crosslinking the anionic polypeptide and the cationic polypeptide of a dried substance obtained by the drying step.

The freezing step can be performed by common methods.

For example, the freezing step can be performed by (a) step of cooling the solution of an anionic polypeptide and a cationic polypeptide under the condition, in which a difference between the temperature of a portion having the highest liquid temperature in the solution and the temperature of a portion having the lowest liquid temperature in the solution is equal to or lower than 2.5°C and the temperature of the portion having the highest liquid temperature in the solution is equal to or lower than the melting point of a solvent, such that the solution becomes in an unfrozen state, and (b) step of freezing the solution of an anionic polypeptide and a cationic polypeptide obtained by the step (a).

At the time of cooling the solution of an anionic polypeptide and a cationic polypeptide so as to make the solution to be in an unfrozen state, the difference between the temperature of a portion having the highest liquid temperature and the temperature of a portion having the lowest liquid temperature in the solution is set to be equal to or lower than 2.5°C (preferably equal to or lower than 2.3°C, and more preferably equal to or lower than 2.1°C), that is, the temperature difference is made small. As a result, the variation in the pore size of the obtained porous substance is reduced. The lower limit of the difference between the temperature of a portion having the highest liquid temperature and the temperature of a portion having the lowest liquid temperature in the solution is not particularly limited, and may be equal to or higher than 0°C. For example, the lower limit may be equal to or higher than 0.1°C, 0.5°C, 0.8°C, or 0.9°C.

It is preferable that the cooling in the step (a) is performed through a material having a thermal conductivity lower than that of water. A portion farthest from the cooling side can be regarded as the portion having the highest liquid temperature in the solution, and the portion having the lowest liquid temperature in the solution can be regarded as having the liquid temperature of the cooling surface.

In the step (a), immediately before the heat of solidification is generated, the difference between the temperature of the portion having the highest liquid temperature in the solution and the temperature of the portion having the lowest liquid temperature in the solution is preferably equal to or lower than 2.5°C, more preferably equal to or lower than 2.3°C, and even more preferably equal to or lower than 2.1°C. "Temperature difference immediately before the heat of solidification is generated" means a temperature difference which becomes the largest for 1 to 10 seconds before the generation of the heat of solidification.

In the step (a), the temperature of the portion having the lowest liquid temperature in the solution is preferably equal to or lower than the melting point of the solvent - 5°C, more preferably equal to or lower than the melting point of the solvent - 5°C and equal to or higher than melting point of the solvent - 20°C, and even more preferably equal to or higher than the melting point of the solvent - 6°C and equal to or higher than the melting point of the solvent - 16°C. The solvent in "melting point of the solvent" is the solvent of the solution of an anionic polypeptide and a cationic polypeptide.

In the step (b), the solution of an anionic polypeptide and a cationic polypeptide obtained by the step (a) is frozen. The cooling temperature for freezing in the step (b) is not particularly limited and depends on the cooling instrument. The cooling temperature is preferably a temperature 3°C to 30°C lower than the temperature of the portion having the lowest liquid temperature in the solution, more preferably a temperature 5°C to 25°C lower than the temperature of the portion having the lowest liquid temperature in the solution, and even more preferably a temperature 10°C to 20°C lower than the temperature of the portion having the lowest liquid temperature in the solution.

The drying step can be performed by common methods. For example, the drying can be performed by conducting vacuum drying at a temperature lower than the melting point of the solvent and then conducting vacuum drying again at room temperature (20°C).

The drying time is not particularly limited, and the drying can be generally performed for about 12 hours to 144 hours. In a case where the vacuum drying at a temperature lower than the melting point of the solvent and the vacuum drying at room temperature (20°C) are performed as described above, for example, the vacuum drying at a temperature lower than the melting point of the solvent can be performed for 6 hours to 48 hours, and the vacuum drying at room temperature (20°C) can be performed for 6 hours to 96 hours.

The crosslinking step of crosslinking the anionic polypeptide and the cationic polypeptide of a dried substance obtained by the drying step can be performed in the manner described above in the present specification.

In the present invention, the shape of the crosslinked substance is not particularly limited. For example, the crosslinked substance has a definite shape, an amorphous shape, a spherical shape, a particle (granule) shape, a powder shape, a porous shape, a fibrous shape, a spindle shape, a flat shape, a sheet shape, and the like. The amorphous shape means a shape that does not have a uniform surface shape, and examples thereof include the shape a stone having irregularities. The shapes exemplified above are not independent from each other. For example, in some cases, the crosslinked substance has the amorphous shape as an example of the subordinate concepts of the particle (granule) shape.

[Method for manufacturing preparation of the present invention]

The present invention also provides a method for manufacturing the preparation of the present invention, including a freezing step of freezing a solution containing an anionic polypeptide and a cationic polypeptide; a drying step of drying a frozen substance obtained by the freezing step; a crosslinking step of crosslinking the anionic polypeptide and the cationic polypeptide of a dried substance obtained by the drying step; and a drug adding step of incorporating a cationic drug into a crosslinked substance of the anionic polypeptide and the cationic polypeptide obtained by the crosslinking step.

The drying step and the crosslinking step can be performed in the manner described above in the present specification.

The drug adding step is not particularly limited as long as a cationic drug can be incorporated into the crosslinked substance. For example, by adding a solution containing the cationic drug to the crosslinked substance and impregnating the crosslinked substance with the solution, the cationic drug can be incorporated into the crosslinked substance.

Hereinafter, the present invention will be more specifically described by the following examples, but the present invention is not limited to the examples.

Examples

[Cationic polypeptide]

As a cationic polypeptide, CBE3 described below was prepared (see WO2008/103041A).
CBE3
Molecular weight: 51.6 kDa
Structure: GAP[(GXY)$_{63}$]$_3$G

Number of amino acids: 571

Number of RGD sequences: 12
Content of imino acid: 33%

Approximately 100% of the amino acids are repeating GXY structures. The amino acid sequence of CBE3 does not contain serine, threonine, asparagine, tyrosine, and cysteine. CBE3 has an ERGD sequence.

Isoelectric point: 9.34
GRAVY value: -0.682
1/IOB value: 0.323

Amino acid sequence (SEQ ID NO: 1 in sequence listing) (same as SEQ ID NO: 3 in WO2008/103041A except that the terminal X is revised to "P")

GAP(GAPGLQGAPGLQGMPGERGAAGLPGPKGERGDAGPKGADGAPGAPGLQGMPG

ERGAAGLPGPKGERGDAGPKGADGAPGKDGVRGLAGPIGPPGERGAAGLPGPKGER

GDAGPKGADGAPGKDGVRGLAGPIGPPGPAGAPGAPGLQGMPGERGAAGLPGPKGE

RGDAGPKGADGAPGKDGVRGLAGPP)3G

[Preparation of succinylated CBE3 as anionic polypeptide]

An aqueous solution containing 6.3% by mass of CBE3 and 1.5% by mass of boric acid and adjusted to have a pH of 10 was prepared, and 100 mL of this aqueous solution was put into a three-neck flask having a volume of 300 mL. Succinic anhydride (0.8% by mass) was added to the aqueous solution in the three-neck flask, and the mixture was reacted for 2 hours while being stirred at 37°C. By using a dialysis membrane having a molecular weight cut off (MWCO) of 3,000, the reaction product was purified by performing dialysis three times against distilled water of an amount 150 times greater than that of the reaction product, thereby obtaining succinylated CBE3. In the following experiment, the succinylated CBE3 was used as an anionic polypeptide.

[Manufacturing of member for preparation formed of crosslinked substance of anionic polypeptide and cationic polypeptide (Example 1)]

A mixed aqueous solution of CBE3 and the succinylated CBE3 was prepared, and 8 mL of this solution was put into a cylindrical container made of polytetrafluoroethylene (PTFE). At this time, the concentration of the polypeptides (total concentration of CBE3 and the succinylated CBE3) was set to be 4% by mass.

CBE3 and the succinylated CBE3 in the aqueous solution were combined such that a ratio therebetween became as shown below.

"A" molar ratio of CBE3 to succinylated CBE3 = 1 to 3
"B" molar ratio of CBE3 to succinylated CBE3 = 1 to 1
"C" molar ratio of CBE3 to succinylated CBE3 = 3 to 1

The cylindrical container made of PTFE containing the mixed aqueous solution was cooled from the bottom surface thereof in a vacuum freeze-drying machine (TF5-85ATNNN: manufactured by TAKARA CO. LTD.) by using a cooling rack. The temperature of the rack was set by cooling the rack to 10°C and freezing the rack for 1 hour at -10°C, then for 2 hours at -20°C, for 3 more hours at -40°C, and finally for 1 hour at -50°C. After the temperature of the rack was set to become -20°C again, the frozen product obtained in this way was vacuum-dried for 24 hours at -20°C. After 24 hours, in a state where the vacuum drying was being continued, the temperature of the rack was increased to 20°C, and the vacuum drying was performed for 48 more hours at 20°C until a degree of vacuum sufficiently decreased (1.9 $\times$ $10^5$ Pa). Thereafter, the product was taken out of the vacuum freeze-drying machine, thereby obtaining a porous substance.

A heat treatment was performed on the porous substance for 24 hours at 135°C in a nitrogen atmosphere such that crosslinking occurred.

[Manufacturing of member for preparation containing anionic polypeptide and cationic polypeptide uncrosslinked (Comparative Example 1)]

A mixed aqueous solution of CBE3 and the succinylated CBE3 was prepared, and 8 mL of this solution was put into a cylindrical container made of PTFE. At this time, the concentration of the polypeptides (total concentration of CBE3 and the succinylated CBE3) was set to be 4% by mass.

CBE3 and the succinylated CBE3 in the mixed aqueous solution were combined such that a ratio therebetween became as shown below.

"D" molar ratio of CBE3 to succinylated CBE3 = 1 to 3
"E" molar ratio of CBE3 to succinylated CBE3 = 1 to 1
"F" molar ratio of CBE3 to succinylated CBE3 = 3 to 1

The cylindrical container made of PTFE containing the mixed aqueous solution was cooled from the bottom surface thereof in a vacuum freeze-drying machine (TF5-85ATNNN: manufactured by TAKARA CO. LTD.) by using a cooling rack. The temperature of the rack was set by cooling the rack to -10°C and freezing the rack for 1 hour at -10°C, then for 2 hours at -20°C, for 3 more hours at -40°C, and finally for 1 hour at -50°C. After the temperature of the rack was set to become -20°C again, the frozen product obtained in this way was vacuum-dried for 24 hours at -20°C. After 24 hours, in a state where the vacuum drying was being continued, the temperature of the rack was increased to 20°C, and the vacuum drying was performed 48 more hours at 20°C until a degree of vacuum sufficiently decreased (1.9 $\times$ $10^5$ Pa). Thereafter, the product was taken out of the vacuum freeze-drying machine, thereby obtaining a porous substance.

[Manufacturing of member for preparation formed of anionic polypeptide or cationic polypeptide (Comparative Example 2)]

An aqueous solution of CBE3 and an aqueous solution of the succinylated CBE3 were prepared, and 8 mL of each of these solutions was put into a cylindrical container made of PTFE. At this time, the concentration of the polypeptide (the concentration of CBE3 or the concentration of the succinylated CBE3) was set to be 4% by mass.

CBE3 and the succinylated CBE3 in the aqueous solution were combined such that a ratio therebetween became as shown below.

"G" molar ratio of CBE3 to succinylated CBE3 = 1 to 0 (the aqueous solution contains only CBE3)

"H" molar ratio of CBE3 to succinylated CBE3 = 0 to 1 (the aqueous solution contains only the succinylated CBE3)

The cylindrical container made of PTFE containing each of the mixed aqueous solutions was cooled from the bottom surface thereof in a vacuum freeze-drying machine (TF5-85ATNNN: manufactured by TAKARA CO. LTD.) by using a cooling rack. The temperature of the rack was set by cooling the rack to -10°C and freezing the rack for 1 hour at -10°C, then for 2 hours at -20°C, for 3 more hours at -40°C, and finally for 1 hour at -50°C. After the temperature of the rack was set to become -20°C again, the frozen product obtained in this way was vacuum-dried for 24 hours at -20°C. After 24 hours, in a state where the vacuum drying was being continued, the temperature of the rack was increased to 20°C, the vacuum drying was performed 48 more hours at 20°C until a degree of vacuum sufficiently decreased (1.9 $\times$ $10^5$ Pa). Thereafter, the product was taken out of the vacuum freeze-drying machine, thereby obtaining a porous substance.

A heat treatment was performed on the obtained porous substance for 24 hours at 135°C in a nitrogen atmosphere such that crosslinking occurred.

[Evaluation of acidolysis properties]

Each of the members for a preparation manufactured in Example 1 and Comparative Examples 1 and 2 was shaped to have a diameter of 5 mm and a thickness of 2 mm.

Two shaped members were added to a 2.0 mL plastic tube whose mass was measured in advance, and the total mass was accurately measured. The mass of the tube was subtracted from the measured mass, thereby determining the mass of a sample before reaction. Then, 1.7 mL of 1 mol/L hydrochloric acid was added thereto and thoroughly mixed together. Thereafter, the 2.0 mL tube was inserted into a heat block (DTU-2B: manufactured by TAITEC CORPORATION) heated to 37°C, and a reaction was performed for 3 hours. After the reaction was finished, the 2.0 mL tube was collected into a foamed polystyrene container containing ice.

The collected 2.0 mL tube was stirred and then subjected to centrifugation for 1 minute at 4°C and 10,000 g. Then, 1,000 $\mu$L of the supernatant was removed, 1,000 $\mu$L of water was added thereto, stirring was performed, and then centrifugation was performed for 1 minute at 4°C and 10,000 g. Thereafter, 900 $\mu$L of the supernatant was removed, 900 $\mu$L of water was added thereto, stirring was performed, and then centrifugation was performed for 1 minute at 4°C and 10,000 g. Furthermore, 900 $\mu$L of the supernatant was removed, 900 $\mu$L of water was added thereto, stirring was performed, and then centrifugation was performed for 1 minute at 4°C and 10,000 g.

In a capped state, the 2.0 mL tube was caused to stand in a tube rack and frozen at -80°C.

After being frozen, the tube rack including the 2.0 mL tube was moved to a vacuum drier (FDU-1000, EYELA) and vacuum-dried.

The 2.0 mL tube was taken out, and the mass thereof was accurately measured. The measured mass is "total mass after reaction" in the following equation.

An acidolysis residual rate was calculated by the following equation. "Tare mass" in the following equation is the mass of the tube.

$$\text{Acidolysis residual rate}(\%) = \frac{\text{total mass after reaction - tare mass}}{\text{sample mass before reaction}} \times 100$$

From the acidolysis residual rate, the decomposition rate of the member in a biological body can be predicted. In order for a drug to be retained for a long period of time in a biological body, it is preferable that the acidolysis residual rate is high (the drug is not easily decomposed). In a case where the acidolysis residual rate is equal to or higher than 50%, the sample was evaluated as excellent, and in a case where the acidolysis residual rate is less than 50%, the sample was evaluated as defective.

The evaluation results of the acidolysis properties of the members prepared in Example 1 and Comparative Examples 1 and 2 are shown in Tables 1, 2, and 3.

[Table 1]

| Example 2: A heat treatment (crosslinking step) was performed. | | | |
|---|---|---|---|
| Member code | A | B | C |
| Molar ratio of succinylated CBE3 to CBE3 (proportion of succinylated CBE3) | 3:1 (75%) | 1:1 (50%) | 1:3 (25%) |
| Evaluation of acidolysis residual rate (actual measurement value) | Excellent (70%) | Excellent (90%) | Excellent (93%) |

[Table 2]

| Comparative Example 1: A heat treatment (crosslinking step) was not performed | | | |
|---|---|---|---|
| Member code | D | E | F |
| Molar ratio of succinylated CBE3 to CBE3 (proportion of succinylated CBE3) | 3:1 (75%) | 1:1 (50%) | 1:3 (25%) |
| Evaluation of acidolysis residual rate (actual measurement value) | Defective (5%) | Defective (6%) | Defective (6%) |

[Table 3]

| Comparative Example 2: A heat treatment (crosslinking step) was performed. | | |
|---|---|---|
| Member code | G | H |
| Molar ratio of succinylated CBE3 to CBE3 (proportion of succinylated CBE3) | 0:1 (0%) | 1:0 (100%) |
| Evaluation of acidolysis residual rate (actual measurement value) | Excellent (98%) | Defective (0%) |

As is evident from the results in Table 1, the member for a preparation formed of the crosslinked substance of CBE3 and succinylated CBE3 had a high acidolysis residual rate and exhibited preferred physical properties.

As is evident from the results in Table 2, in a case where the manufacturing method did not include the crosslinking step, the acidolysis residual rate was low, and preferred physical properties were not exhibited.

As is evident from the results in Table 3, the member for a preparation formed only of the succinylated CBE3 had a low acidolysis residual rate and did not exhibit preferred physical properties. In contrast, the member for a preparation formed only of CBE3 had a high acidolysis residual rate and exhibited preferred physical properties.

[Evaluation of lysozyme carrying rate]

By using an Alexa Fluor 488 Protein Labeling Kit (ThermoFisher Scientific), 1 mg of lysozyme was labeled with fluorescence.

The members for a preparation "A", "B", and "C" manufactured in Example 2 that were formed of the crosslinked substance of an anionic polypeptide and a cationic polypeptide and the member for a preparation "G" manufactured in Comparative Example 2 in which the molar ratio of CBE3 to succinylated CBE3 was 1 to 0 were punched using a biopsy punch having a diameter of 5 mm and then shaped by rounding off the top and bottom thereof, thereby obtaining 2.5 mg of sponge samples.

The members for a preparation "D", "E", an "F" manufactured in Comparative Example 1 that were formed of an anionic polypeptide and a cationic polypeptide that were uncrosslinked and the member "H" of Comparative Example 2 in which the molar ratio of CBE3 to succinylated CBE3 was 0 to 1 were defective because the acidolysis residual rate thereof did not satisfy the standard. Therefore, these members were not evaluated.

Each of the samples was put into a 1.5 ml plastic tube (PROTEOSAVE SS: Sumitomo Bakelite Co., Ltd.), 25 μl of the fluorescence-labeled lysozyme adjusted to have a concentration of 1 mg/ml was added to each of the samples, and the samples were allowed to stand overnight in a refrigerator.

Then, 500 μl of phosphate-buffered physiological saline diluted 10X with ultrapure water was added to each of the samples.

From each of the samples, 10 μl of supernatant was separated immediately after the addition of the saline, after 1 hour, after 3 hours, and after 7 hours, and added to a 96-well black plate.

Phosphate-buffered physiological saline (190 μl) diluted 10X with ultrapure water was added to the separated samples, and the amount of fluorescence thereof was evaluated using a plate reader.

A calibration curve of the fluorescent lysozyme was created, and the amount of the fluorescent lysozyme in the separated samples was calculated.

A lysozyme carrying rate at each time point was calculated as below.

$$\text{Lysozyme carrying rate}(\%) = \frac{\text{amount of lysozyme added } (25\,\mu g) - \text{amount of lysozyme in separated sample} \times \text{amount of supernatant } (525\,\mu l)}{\text{amount of lysozyme added}(25\,\mu g)} \times 100$$

Fig. 1 shows the lysozyme carrying rate of each of the preparations at each time point. The lysozyme carrying rate was stabilized after 3 hours. Accordingly, the samples were compared to each other based on the lysozyme carrying rate at the time point after 3 hours. The results are shown in Tables 4 and 5.

Lysozyme is a form of cationic drug. From the lysozyme carrying rate, the interaction between each of the members for a preparation and a cationic drug can be predicted. The higher the lysozyme carrying rate of a preparation, the stronger the interaction between the preparation and the cationic drug, and hence the preparation is preferable as a slow-release member of the cationic drug. In the present invention, a lysozyme carrying rate equal to or higher than 30% was evaluated as excellent, and a lysozyme carrying rate less than 30% was evaluated as defective.

[Table 4]

| Example 2: A heat treatment (crosslinking step) was performed. | | | |
|---|---|---|---|
| Member code | A | B | C |
| Molar ratio of succinylated CBE3 to CBE3 (proportion of succinylated CBE3) | 3:1 (75%) | 1:1 (50%) | 1:3 (25%) |
| Evaluation of lysozyme carrying rate (actual measurement value) | Excellent (71%) | Excellent (66%) | Excellent (38%) |

[Table 5]

| Comparative Example 2: A heat treatment (crosslinking step) was performed. | |
|---|---|
| Member code | G |
| Molar ratio of succinylated CBE3 to CBE3 (proportion of succinylated CBE3) | 0:1 (0%) |
| Evaluation of lysozyme carrying rate (actual measurement value) | Defective (8%) |

As is evident from the results in Table 4, the member for a preparation formed of the crosslinked substance of CBE3 and succinylated CBE3 had a high lysozyme carrying rate and exhibited preferred physical properties as a slow-release member of a cationic drug.

As is evident from the results in Table 5, the member for a preparation formed only of CBE3 had a low lysozyme carrying rate and did not exhibit preferred physical properties as a slow-release member of a cationic drug.

[Summary]

The evaluation results of the acidolysis residual rate and the lysozyme carrying rate described above are summarized below.

[Table 6]

| Molar ratio of succinylated CBE3 to CBE3 (proportion of succinylated CBE3) | 0:1 (0%) | 1:3 (25%) | 1:1 (50%) | 3:1 (75%) | 1:0 (100%) |
|---|---|---|---|---|---|
| Acidolysis residual rate | A (98%) | A (93%) | A (90%) | B (70%) | C (0%) |
| Lysozyme carrying rate | C (8%) | B (38%) | A (66%) | A (71%) | - |

The acidolysis residual rate was evaluated as A in a case where it was equal to or higher than 90%, evaluated as B in a case where it was equal to or higher than 50% and less than 80%, and evaluated as C in a case where it was less than 50%.

The lysozyme carrying rate was evaluated as A in a case where it was equal to or higher than 50%, evaluated as B in a case where it was equal to or higher than 25% and less than 50%, and evaluated as C in a case where it was less than 25%.

It was revealed that by the constitutions of the present invention, both an excellent acidolysis residual rate and an excellent lysozyme carrying rate can be accomplished.

Sequence Listing

International application 16F02100 accepted based on Patent Cooperation Treaty Preparation, Member for preparation, and JP17012540 20170328----00090016951700672379normal2017032811212420170302115413798O P1AP10 1 16 8.app

## Claims

1. A preparation comprising:

   a crosslinked substance of an anionic polypeptide and a cationic polypeptide; and
   a cationic drug.

2. The preparation according to claim 1 that is for local administration.

3. The preparation according to claim 1 or 2,

   wherein a molar ratio of the anionic polypeptide to the cationic polypeptide is 1 to 3 to 3 to 1.

4. The preparation according to any one of claims 1 to 3,

   wherein each of the anionic polypeptide and the cationic polypeptide is a gene recombinant, which has an amino acid sequence derived from a partial amino acid sequence of collagen, or a chemical modification product thereof.

5. The preparation according to claim 4,

wherein the anionic polypeptide is a chemical modification product of a cationic polypeptide, and the cationic polypeptide is a chemical modification product of an anionic polypeptide.

6. The preparation according to claim 4 or 5,

wherein the gene recombinant having an amino acid sequence derived from a partial amino acid sequence of collagen has a repeating sequence represented by Gly-X-Y, X and Y each independently represent any amino acid,

a plurality of sequences represented by Gly-X-Y may be the same as or different from each other, and a molecular weight of the gene recombinant is equal to or greater than 10 kDa and equal to or smaller than 90 kDa.

7. The preparation according to any one of claims 4 to 6,

wherein the gene recombinant having an amino acid sequence derived from a partial amino acid sequence of collagen is represented by the following formula,

Formula: A-[(Gly-X-Y)n]m-B

in the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.

8. The preparation according to any one of claims 4 to 7,

wherein the gene recombinant having an amino acid sequence derived from a partial amino acid sequence of collagen has (1) amino acid sequence described in SEQ ID NO: 1 or (2) amino acid sequence which shares a sequence identity equal to or higher than 80% with the amino acid sequence described in SEQ ID NO: 1 and has a function of carrying the cationic drug.

9. The preparation according to any one of claims 1 to 8,

wherein the crosslinked substance of an anionic polypeptide and a cationic polypeptide is a crosslinked substance obtained by a heat treatment.

10. The preparation according to any one of claims 1 to 9,

wherein the cationic drug is a polypeptide.

11. A member for a preparation comprising:

a crosslinked substance of an anionic polypeptide; and
a cationic polypeptide.

12. The member for a preparation according to claim 11,

wherein a molar ratio of the anionic polypeptide to the cationic polypeptide is 1 to 3 to 3 to 1.

13. The member for a preparation according to claim 11 or 12,

wherein each of the anionic polypeptide and the cationic polypeptide is a gene recombinant, which has an amino acid sequence derived from a partial amino acid sequence of collagen, or a chemical modification product thereof.

14. The member for a preparation according to claim 13,

wherein the anionic polypeptide is a chemical modification product of a cationic polypeptide, and the cationic polypeptide is a chemical modification product of an anionic polypeptide.

**15.** The member for a preparation according to claim 13 or 14,

wherein the gene recombinant having an amino acid sequence derived from a partial amino acid sequence of collagen has a repeating sequence represented by Gly-X-Y, X and Y each independently represent any amino acid,

a plurality of sequences represented by Gly-X-Y may be the same as or different from each other, and

a molecular weight of the gene recombinant is equal to or greater than 10 kDa and equal to or smaller than 90 kDa.

**16.** The member for a preparation according to any one of claims 13 to 15,

wherein the gene recombinant having an amino acid sequence derived from a partial amino acid sequence of collagen is represented by the following formula,

Formula:          A-[(Gly-X-Y)n]m-B

in the formula, A represents any amino acid or any amino acid sequence, B represents any amino acid or any amino acid sequence, n X's each independently represent any amino acid, n Y's each independently represent any amino acid, n represents an integer of 3 to 100, m represents an integer of 2 to 10, and n sequences represented by Gly-X-Y may be the same as or different from each other.

**17.** The member for a preparation according to any one of claims 13 to 16,

wherein the gene recombinant having an amino acid sequence derived from a partial amino acid sequence of collagen has (1) amino acid sequence described in SEQ ID NO: 1 or (2) amino acid sequence which shares a sequence identity equal to or higher than 80% with the amino acid sequence described in SEQ ID NO: 1 and has a function of carrying the cationic drug.

**18.** The member for a preparation according to any one of claims 11 to 17,

wherein the crosslinked substance of an anionic polypeptide and a cationic polypeptide is a crosslinked substance obtained by a heat treatment.

**19.** A method for manufacturing the member for a preparation according to any one of claims 11 to 18, comprising:

a freezing step of freezing a solution containing an anionic polypeptide and a cationic polypeptide;

a drying step of drying a frozen substance obtained by the freezing step; and

a crosslinking step of crosslinking the anionic polypeptide and the cationic polypeptide of a dried substance obtained by the drying step.

**20.** A method for manufacturing the preparation described in any one of claims 1 to 10, comprising:

a freezing step of freezing a solution containing an anionic polypeptide and a cationic polypeptide;

a drying step of drying a frozen substance obtained by the freezing step;

a crosslinking step of crosslinking the anionic polypeptide and the cationic polypeptide of a dried substance obtained by the drying step; and

a drug adding step of incorporating a cationic drug into a crosslinked substance of the anionic polypeptide and the cationic polypeptide obtained by the crosslinking step.

## FIG. 1

----◇---- MOLAR RATIO OF SUCCINYLATED CBE3:CBE3 = 0 : 1
(COMPARATIVE EXAMPLE)

——▲—— MOLAR RATIO OF SUCCINYLATED CBE3:CBE3 = 1 : 3

——●—— MOLAR RATIO OF SUCCINYLATED CBE3:CBE3 = 1 : 1

——✕—— MOLAR RATIO OF SUCCINYLATED CBE3:CBE3 = 3 : 1

**INTERNATIONAL SEARCH REPORT**

| International application No. |
|---|
| PCT/JP2017/012540 |

**A. CLASSIFICATION OF SUBJECT MATTER**
*A61K47/42*(2017.01)i, *A61K38/46*(2006.01)i, *A61K45/00*(2006.01)i

According to International Patent Classification (IPC) or to both national classification and IPC

**B. FIELDS SEARCHED**

Minimum documentation searched (classification system followed by classification symbols)
A61K47/00-47/69, A61K9/00-9/72, A61K47/42, A61K45/00, A61K38/00-38/58, A61K31/00-33/44

Documentation searched other than minimum documentation to the extent that such documents are included in the fields searched
Jitsuyo Shinan Koho          1922-1996   Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2006-307004 A  (Osaka University),<br>09 November 2006 (09.11.2006),<br>claims; paragraphs [0039], [0042]<br>(Family: none) | 11,12<br>1-10,13-20 |
| Y | JP 2010-519253 A  (Fujifilm Manufacturing Europe B.V.),<br>03 June 2010 (03.06.2010),<br>claims; paragraphs [0028], [0032], [0046], [0057]<br>& US 2010/0203138 A1<br>claims; paragraphs [0037], [0041], [0061], [0075]<br>& WO 2008/103045 A1 | 1-10,13-20 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    13 June 2017 (13.06.17) | Date of mailing of the international search report<br>    27 June 2017 (27.06.17) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2017/012540

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2010-520860 A (Fujifilm Manufacturing Europe B.V.), 17 June 2010 (17.06.2010), claims; paragraphs [0039], [0046], [0053] & US 2010/0048481 A1 claims; paragraphs [0039], [0046], [0056] & WO 2008/103046 A1 | 1-10,13-20 |
| Y | JP 2010-031003 A (Nitto Denko Corp.), 12 February 2010 (12.02.2010), paragraphs [0007], [0026] to [0030] & US 2011/0189299 A1 paragraphs [0012], [0041] to [0045] & WO 2010/001932 A1 & EP 2308473 A1 | 1-10,20 |
| Y | JP 2004-277348 A (Medgel Corp.), 07 October 2004 (07.10.2004), paragraphs [0013], [0018] & US 2006/0251719 A1 paragraphs [0020], [0025] & WO 2004/082657 A1 & EP 1607085 A1 | 19,20 |
| A | JP 2012-532839 A (Marina Biotech, Inc.), 20 December 2012 (20.12.2012), claims & US 2012/0237589 A1 claims & WO 2011/003834 A1 & EP 2277508 A1 | 1-20 |
| A | JP 2006-299026 A (Toppan Printing Co., Ltd.), 02 November 2006 (02.11.2006), claims (Family: none) | 1-20 |
| A | US 2010/0209518 A1 (GEORGIEVA, Radostina), 19 August 2010 (19.08.2010), claims & WO 2009/010494 A2 & EP 2014280 A1 | 1-20 |
| A | WO 2014/199982 A1 (University of Tsukuba), 18 December 2014 (18.12.2014), claims & US 2016/0175247 A1 claims & EP 3009140 A1 | 1-20 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003048841 A
- JP 2006096751 A
- WO 2015025979 A
- EP 1014176 A
- US 6992172 A
- WO 200485473 A
- WO 2008103041 A
- EP 1014176 A2
- JP 17012540 A

**Non-patent literature cited in the description**

- Pathophysiology. Nagai Publishing Co., Ltd, 1990, vol. 9, 527
- **MAXEY, C. R.** Phintogr. Gelatin 2. P. J. Academic, 1976
- *J. Mol. Biol.,* 1990, vol. 215, 403-410
- *Pharmaceutical Bulletin,* 1954, vol. 2 (2), 163-173
- *Scope of Chemistry,* 1957, vol. 11 (10), 719-725
- *Fragrance Journal,* 1981, vol. 50, 79-82
- **YOSHIKI KODA et al.** New Edition of Organic Conception Diagram-Fundamentals and Applications. SANKYO SHUPPAN Co., Ltd, 2008
- Protein Identification and Analysis Tools on the ExPASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788